Europäisches Patentamt

European Patent Office

Office européen des brevets

(19)

(11) Publication number: **0 119 864**
**B1**

# EUROPEAN PATENT SPECIFICATION

(45) Date of publication of patent specification: **26.10.88**

(51) Int. Cl.⁴: **A 61 M 15/02, A 61 B 5/08**

(21) Application number: **84301879.7**

(22) Date of filing: **20.03.84**

(54) Radioaerosol delivery apparatus and manifold therefor.

(30) Priority: **21.03.83 US 477277**
**21.03.83 US 477276**

(43) Date of publication of application:
**26.09.84 Bulletin 84/39**

(45) Publication of the grant of the patent:
**26.10.88 Bulletin 88/43**

(84) Designated Contracting States:
**AT BE CH DE FR GB IT LI LU NL SE**

(56) References cited:
**EP-A-0 069 104**
**US-A-3 881 463**

**ATOMKERNENERGIE (ATKE), vol. 26, no. 2,
1975, pages 125-128, Verlag K. THIEMIG A.G.,
Munich, DE; W.M. PUSCH: "Ein
Versuchsaufbau für Inhalationsexperimente
mit radioaktiven Aerosolen"**

(73) Proprietor: **MALLINCKRODT, INC.**
**P.O. Box 5840 675 McDonnell Boulevard
St. Louis, State of Missouri (US)**

(72) Inventor: **Iannuzzelli, Vincent F.
Rte 3 Box 115 Big Spring Road
Califon New Jersey 07830 (US)**
Inventor: **Kremer, Carl P.
8 Rings End Road
Darien Connecticut 06280 (US)**
Inventor: **Burnett, Thomas W.
4691 148th Place S.E.
Bellevue Washington 98006 (US)**
Inventor: **Clary, Thomas R.
234 N.W. 55th Street
Seattle Washington 98107 (US)**

(74) Representative: **Armitage, Ian Michael et al
MEWBURN ELLIS & CO. 2/3 Cursitor Street
London EC4A 1BQ (GB)**

(56) References cited:
**JOURNAL OF NUCLEAR MEDICINE, vol. 15, no.
4, April 1974, pages 288-290; R.H. SECKER-
WALKER et al.: "A simple 133Xe delivery
system for studies of regional ventilation"**

## Description

### BACKGROUND OF THE INVENTION
#### Field of the Invention

This invention relates to apparatus for delivery of radioactive materials. In particular, the invention relates to shielding apparatus and manifold utilized with radioaerosol delivery systems in nuclear medicine.

#### State of the Art

Lung ventilation scanning using radiolabled aerosols has been studied for about the last 20 years. However, until recently when improved aerosol generating devices have become more generally available, practical applications of such methods have been extremely limited. One particularly useful aerosol generating system is that described in U.S. Patent No. 4,116,387 and U.S. Patent No. 4,251,033. The nebulizer described in those patents has been found to be particularly useful in generating aerosols having a particle size and particle size distribution to make lung scanning a useful diagnostic tool. Relatively recent articles describing lung scanning methodology utilizing radioactive aerosols can be found at: Radiology, 131:256-258, April 1979; Seminars in Nuclear Medicine, Volume X, No. 3 (July), 1980, pp. 243-251; and The Journal of Nuclear Biology and Medicine, Vol. 19, No. 2, 1975, pp. 112-120.

Because of the increased interest in using radioaerosols for diagnostic imaging, there is a need for a compact and practical apparatus for delivering such radioaerosols to a patient.

### SUMMARY OF THE INVENTION

The present invention is directed in one aspect to an apparatus comprising support means for supporting a radioaerosol generating source; transport means connectable to the source for transporting a radioactive aerosol generated by the source to a patient in fluid communication with the source; and shielding means substantially surrounding the support means and the transport means for reducing the amount of radiation transmitted to the surroundings, a portion of the shielding means being releasably attachable to the transport means and being removable with said transport means and said source from the support means as a unit.

In another aspect, the invention is directed to a shielding container comprising an outer shell; an inner shell supported within the outer shell, the inner shell being formed with an inner wall and an outer wall defining a space therebetween for receiving radiation shielding material, the inner wall having a portion thereof conforming substantially to the contours of a radioaerosol source and transport means to be placed therein; and a removable cover formed with radiation shielding material and having a portion thereof conforming generally to the contours of the radioaerosol transport means to be positioned thereunder, the inner wall and the cover defining at least one opening therebetween to permit the transport means placed therein to be in fluid communication with the surrounding atmosphere and/or patient when in use.

In still another aspect the invention is directed to a manifold comprising a first, rigid conduit and a second, rigid conduit joined at one end to form a first connector and at the other end to form a second connector; a third connector located in the first conduit between the first and second connector; a first, one-way valve located in the first conduit between the first connector and the third connector permitting fluid flow through the first conduit in a direction from the first connector toward the second connector; and a second, one-way valve located in the second conduit permitting fluid flow through the second conduit in a direction from the second connector toward the first connector and preventing flow in the reverse direction. In a presently preferred embodiment the two conduits define an opening between them and have an attachment means formed thereon to attach the manifold to a manifold support means. Additionally, the third connector on the manifold is provided with a locking groove to engage a complementary ring in the mouth of a nebulizer.

### DETAILED DESCRIPTION OF THE DRAWINGS

Figure 1 is a perspective view of the shielding container;

Figure 2 is a front, sectional view of the shielding container;

Figure 3 is a front, sectional view of the shielding container including the radioaerosol source generator and transport means positioned within the container;

Figure 4 is a top view of the shielding container with the lid removed illustrating the surface configuration of the inner shell of the container;

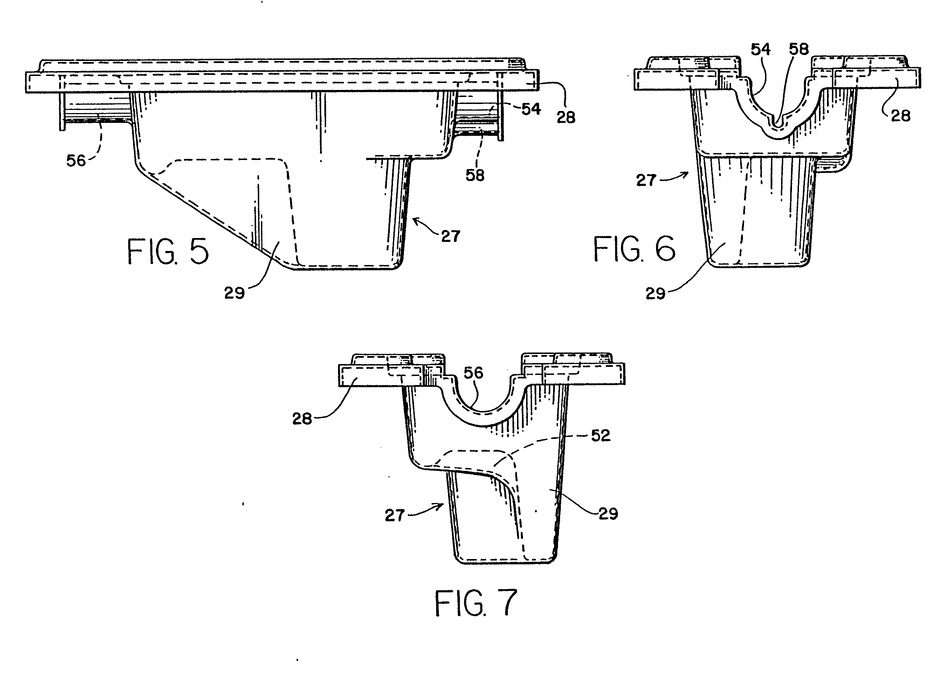

Figure 5 is a side view of the inner shell;

Figure 6 is a view of one end the inner shell of Figure 5;

Figure 7 is a view of the other end of the inner shell of Figure 5;

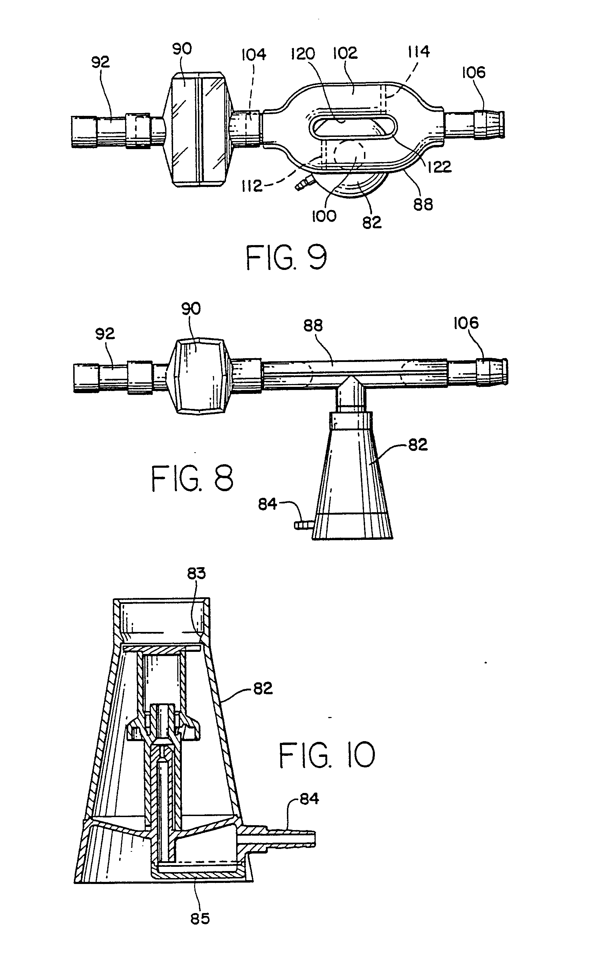

Figure 8 is a side view of the manifold utilized to transport the radioaerosol and the nebulizer connected thereto;

Figure 9 is a top view of the manifold and nebulizer illustrated in Figure 8;

Figure 10 is cross-sectional view of a nebulizer utilized with the invention;

Figure 11 is an exploded view of the manifold illustrating the component parts; and

Figure 12 is a side view of the cover shield portion of the shielding container with the transport means and the radioaerosol source generator connected.

### DETAILED DESCRIPTION OF THE INVENTION

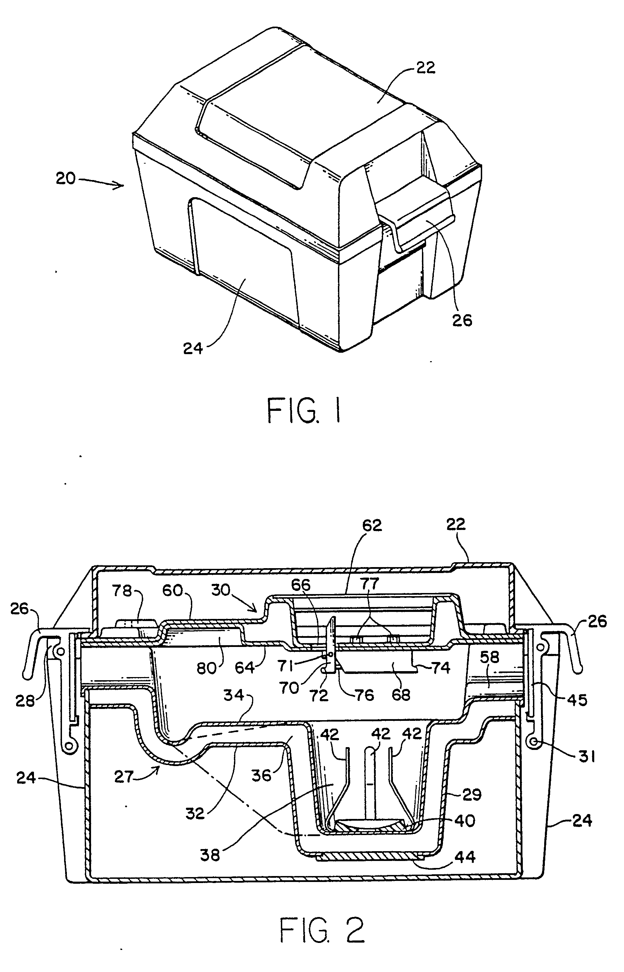

The shielding apparatus and container 20 is illustrated generally in Figure 1. Container 20 has an outer shell 24 upon which is located a closure lid 22. Lid 22 is hingedly connected to outer shell 24 and can be secured to outer shell 24 by handle members 26 (one of which is illustrated) which are also hingedly connected to the outer shell 24. The members 26 function both as handles and as a closure means for the container.

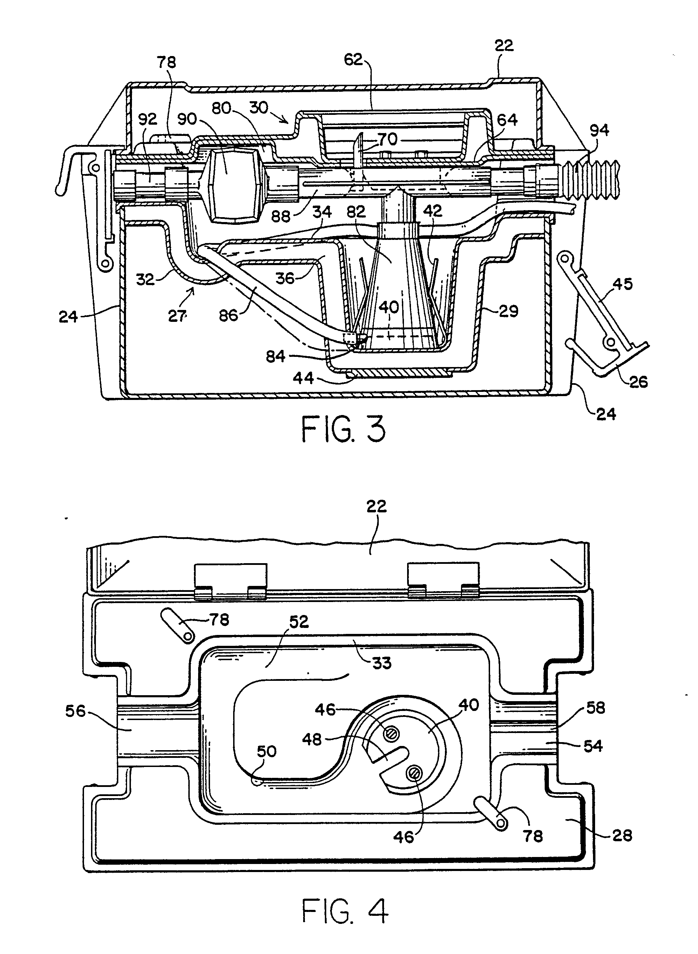

As can best be seen in Figures 2 and 4, container 20 is provided with an inner shell 27 which is supported on and in outer shell 24. Inner shell 27 is formed with a top portion 28 which is adapted to be bonded to outer shell 24 around the periphery thereof. Inner shell 27 additionally has a lower portion 29 formed by outer wall 32 and inner wall 34. A cover shield 30, which will be described more fully hereinafter, is adapted to fit within the surfaces defined by inner wall 34 of the lower portion 29. Outer wall 32 and inner wall 34 define a channel 36 therebetween which can be filled with a suitable radiation shielding material (not shown) such as lead pellets or the like. Inner wall 34 defines a nebulizer well 38 which generally conforms to the contours of the nebulizer 82 when it is located within well 38.

A support pad 40 is provided at the bottom of well 38 and secured thereto by means of screws 46 which can be seen most clearly in Figure 4. A radial slot 48 is formed in the support pad 40 to accommodate the bottom portion 85 of nebulizer 82. Also provided in nebulizer well 38 are retaining spring elements 42 which are suitably formed from spring steel or the like and are adapted to contact the wall of nebulizer 82 to maintain it in a stable and upright position during use. Attached to the bottom of outer wall 32 is a plate 44 which is utilized to cover the opening through which lead pellets or other suitable shielding material can be loaded into channel 36. Alternatively, the shielding material can be placed within channel 36 during the molding process. Inner wall 34 is contoured and includes a ramp sidewall 50 which defines a ramp 52 extending about the periphery of inner wall 34 from the bottom of well 38 to the top of well 38 and eventually to groove 58 in the hemicylindrical surface 54 formed at one end of the inner shell 27. Ramp 52 is utilized to support a fluid delivery tube 86 which extends from inlet port 84 on nebulizer 82 upwardly upon ramp 52 through groove 58 where it can be attached to a source of air or oxygen to drive nebulizer 82 in a conventional manner. Ramp 52 provides a convenient mechanism for ensuring that the fluid delivery tube 86 does not kink or become unduly twisted and thus prevent fluid delivery and operation of the nebulizer 82.

Handles 26 are hingedly connected at pivot points 31 to outer shell 24 and are adapted to engage lid 22 in the closed position. Only one hinge mechanism has been illustrated but it is understood that handle 26 on the other side of container 20 is connected in the same fashion. Handle 26 is additionally provided with radiation shielding material 45 in the form of a lead plate or the like.

Inner wall 34 also defines a support surface 33 about the periphery of inner shell 27 dimensioned to mate with cover shield 30, which is formed with the same design about its periphery. Cover shield 30 is formed with a contoured top plate 60 on which is mounted a handle 62. Attached to the bottom of top plate 60 is a contoured shield plate 64 which is made from radiation shielding material. Both top plate 60 and shield plate 64 are formed with a hole 66 extending therethrough to accommodate a movable latch 70 which is utilized to engage the manifold 88.

Movable latch 70 is pivotably attached to a latch support 68 at pivot point 71. Latch 70 is formed with a surface 72 and latch support 68 is formed with a surface 74 which are adapted to engage a portion of the manifold 88. Latch surface 72 is movable, whereas latch surface 74 remains fixed. A spring-loaded latch rod 76 is provided between latch 70 and latch support 68 in order to bias latch 70 to its engaged position. Latch rod 76 is conveniently located within a bore formed in latch support 68. Latch support 68 is conveniently attached to cover shield 30 by means of screws 77. Means to retain cover shield 30 are provided by means of pivotable arms 78 which are connected to the top portion 28 of inner shell 27 and adapted to be moved over the cover shield 30 when it is in position. The contours of cover shield 30 define a filter well 80 which is adapted to accommodate filter 90 when it is in place as part of the transport means for the radioaerosol, as illustrated most clearly in Figure 3.

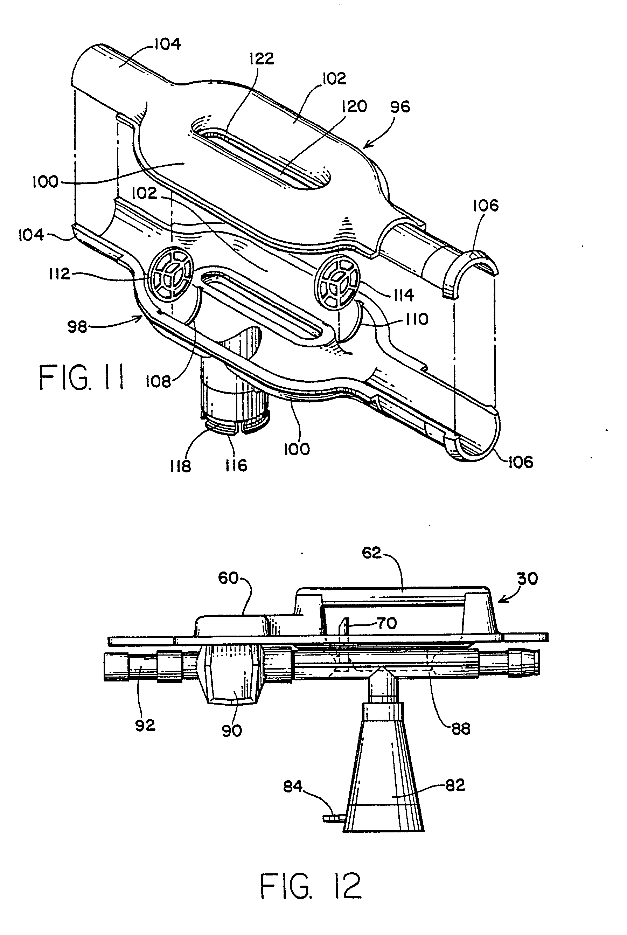

Figure 3 illustrates generally the relative positions of the various components of the apparatus when the radioaerosol system is in use. As can be seen therein the nebulizer 82 is positioned on support pad 40 in well 38 and retained by spring members 42 in a stable and upright position. The lower end of the nebulizer 82 is provided with a connector 84 which is adapted to receive the end of a fluid supply tube 86 which is supported on ramp 52 and directed through the groove 58 formed in inner shell 27. Fluid supply tube 86 is connected to a source of air or oxygen to drive the nebulizer in a conventional manner. The top of nebulizer 82 is formed with a molded, inner ring 83 which is adapted to locate within a groove 118 on a connector 116 at the bottom of the manifold 88, as can be seen most clearly in Figure 11. The nebulizer 82 is connected to the manifold via connector 116 and the manifold 88 is engaged by movable latch 70 and thus is secured to cover shield 30.

End 104 of manifold 88 is connected to a biological filter 90 and the other end 106 of manifold 88 is connected to a patient breathing tube 94 which extends to the mouthpiece of the patient. An extension 92 is placed on the end of filter 90 to assist in the support of the transport means within the shielding container. When cover shield 30 is attached to manifold 88, as can best be seen in Figure 12, cover shield 30 and the transport means (including manifold 88, filter 90 and filter extension 92) and the radioaerosol generating source, i.e., the nebulizer 82, can be removed from the shielding container as a unit. Thus, in removing that system as a unit from the shielding container, the operator still is protected

by cover shield 30 in handling the manifold 88, filter 90 and nebulizer 82 and associated tubing which may be contaminated with radioactive material. The entire unit can then be placed over a suitable disposal container and when latch 70 is pivoted to release manifold 88, nebulizer 82, filter 90 and associated tubing also are released so that all of the contaminated components will be disposed of without unduly endangering an operator.

As can be seen most clearly in Figure 11, the manifold 88 is formed with an upper section 96 and a lower section 98 which when joined together form an inlet conduit 100 and an outlet conduit 102 which join at one end to form a connector 104 which is adapted to connect to the filter 90 and at the other end form a connector 106 which is adapted to connect to the patient breathing tube 94. Inlet conduit 100 and outlet conduit 102 define an opening 120 which is provided with a lip 122 extending outwardly from conduit 100 and 102 into the opening. The function of lip 122 is to be engaged by surfaces 72 and 74 on the latching mechanism. A one-way check valve 112 is situated in a groove 108 formed in inlet conduit 100 between connector 104 and connector 116. Valve 112 is conventional and can be of the diaphragm type. Valve 112 permits flow from the atmosphere through the filter from connector 104 in a direction toward connector 106 through inlet conduit 100. However, the one-way nature of valve 112 will prevent fluid flow in the reverse direction, for example when the patient exhales. In a similar manner, a one-way valve 114 is provided in a groove 110 in outlet conduit 102. One-way valve 114 can again be of the diaphragm type and will permit flow in a direction from connector 106 through outlet conduit 102 to connector 104. Valve 114 will, however, prevent flow in the opposite direction. As described above, latch support 68 and latch 70 are adapted to fit within opening 120 such that surfaces 72 and 74 can engage the lower portion of lip 122 formed on inlet conduit 100 and outlet conduit 102. While lip 122 extends entirely around the periphery of opening 120, it is understood that only portions thereof would have to be provided in order to attach manifold 88 to cover shield 30.

In the event it is not appropriate to dispose of the transport means and the nebulizer 82 immediately after use, the patient tube 94 and the fluid delivery tube 86 can be disconnected and handles 26 can be moved upwardly and latched to lid 22 to position radiation shielding material 45 over the ends of the openings (54,56) in the outer shell at each side of the container. Thus the container 20 effectively isolates the radioactive material from the surrounding atmosphere and the radioactive material can be left within container 20 until such time as the level of radioactivity has been reduced to a point that the disposal is appropriate.

During operator use, lid 22 is elevated and an aerosol generator, such as nebulizer 82 is connected to the fluid delivery tube 86 and positioned within the bottom of well 38 upon support pad 40.

Tube 86 is supported on ramp 52 and directed through groove 58. A radiolabeled solution such as 99m technetium diethylenetriaminepentaacetate or sulphur colloid in a shielded syringe in a conventional manner is dispensed into nebulizer 82. Then the manifold 88 connected to cover shield 30 and the filter 90 and associated tubing are positioned above nebulizer 82 and inserted in the contour formed by inner wall 34 onto support surface 33. By pushing downwardly on cover shield 30, which is connected to manifold 88, connector 116 of manifold 88 is forced into the upper end of nebulizer 82 and groove 118 and ring 83 engage to secure the nebulizer 82 to manifold 88. The patient tubing 94 can than be attached to end 106 of manifold 88, unless it was attached beforehand.

After connection of fluid delivery tube 86 to a source of driving fluid for nebulizer 82, the inhalation process can proceed in a conventional manner. As the patient inhales, the patient breathes radiolabeled aerosol generated from nebulizer 82. In the event the fluid flow volume is insufficient to satisfy the inhalation volume requirement of the patient, additional air will be brought in from the atmosphere through filter extension 92, filter 90, valve 112 and through inlet conduit 100. In that manner the patient does not feel uncomfortable if the aerosol flow volume is too low to satisfy his demands. When the patient exhales, the expired gases pass through valve 114 and outlet conduit 102, where any radioactive substance is collected by filter 90. At the end of the procedure, the flow of drive fluid to the nebulizer is ended and the patient is removed from the unit. At that time the filter, manifold 88 and nebulizer 82 can be removed from the container 20 as a unit for immediate disposal or, as has been described previously, handles 26 can be pivoted upwardly to latch to lid 22 and close the end openings through which the fluid transport system communicated with the atmosphere and the patient.

**Claims**

1. Radioaerosol delivery apparatus comprising support means (40,42) for supporting a radioaerosol generating source (82); transport means (88) connectable to said source (82) for transporting a radioaerosol generated by said source to a patient in fluid communication with said source; and shielding means (30-36) substantially surrounding said support means and said transport means for reducing the amount of radiation transmitted to the surroundings, characterised in that a portion (30) of said shielding means is releasably attachable to said transport means (88) and is removable with said transport means and said source (82) from said support means (40,42) as a unit.

2. The apparatus of claim 1 wherein said transport means comprises a manifold (88) including first attachment means (122) adapted for releasable attachment to said portion (30) of said shielding means.

3. The apparatus of claim 2 wherein said portion (30) of said shielding means includes second attachment means (68-76) for releasably engaging said first attachment means (122).

4. The apparatus of any one of claims 1, 2 and 3 wherein said manifold is formed as a rigid unit and includes a first conduit (100) and a second conduit (102) joined at one end to form a first connector (104) and at the other end to form a second connector (106): and a third connector (116) located in said first conduit (100) between said first and second connectors and adapted for attachment to said source (82).

5. The apparatus of claim 4 wherein said first and second conduits (100,102) define an opening (120) between them and first attachment means (122) adapted for releasable attachment to said portion (30) of said shielding means are formed on at least one of said conduits.

6. The apparatus of claim 5 wherein said portion (30) of said shielding means includes second attachment means (68-76) adapted to enter said opening (120) and releasably engage said first attachment means (122).

7. The apparatus of claim 6 wherein said first attachment means includes a lip (122) and said second attachment means includes at least one surface (72) for engaging said lip.

8. The apparatus of any one of claims 4 to 7 including a first, one-way valve (112) located in said first conduit (100) between said first connector (104) and said third connector (116) permitting fluid flow through said first conduit (100) in a direction from said first connector (104) towards said second connector (106) and preventing fluid flow in the reverse direction and a second one-way valve (114) located in said second conduit (102) permitting fluid flow through said second conduit in a direction from said second connector (106) towards said first connector (104) and preventing fluid flow in the reverse direction.

9. The apparatus of any one of the preceding claims wherein said shielding means includes a shielding container comprising:

an outer shell (24);

an inner shell (27) supported within said outer shell, said inner shell being formed with an inner wall (34) and outer wall (32) defining a space (36) therebetween for receiving radiation shielding material, said inner wall (34) having a portion thereof conforming generally to the contours of a radioaerosol source and transport means to be placed therein; and

a removable cover (30) formed with radiation shielding material and having a portion thereof conforming generally to the contours · of the radioaerosol transport means to be positioned thereunder, said inner wall and said cover defining at least one opening (54,56) between them to permit the transport means placed therein to be in fluid communication with the surrounding atmosphere when in use.

10. The apparatus of claim 9 wherein said inner wall (34) defines a ramp (52) adapted to support a fluid delivery tube to the radioaerosol source.

11. The apparatus of any one of the preceding claims, incorporating at least one opening (54,56) to permit the transport means placed therein to be in fluid communication with the surrounding atmosphere when in use; and auxiliary radiation shielding means (26,45) to close said opening or openings (54,56) when said source and transport means is not in use to effectively isolate said source and transport means from the surrounding atmosphere.

12. The apparatus of claim 11 including a lid (22) adapted to be retained by said auxiliary shielding means (26,45) when said auxiliary shielding means are positioned to close said opening or said openings (54,56).

13. A manifold adapted for use in the transport means of the apparatus of claim 1 comprising a first rigid conduit (100) and a second rigid conduit (102) joined at one end to form a first connector (104) and at the other end to form a second connector (106);

a third connector (116) located in said first conduit (100) between said first and second connectors (104,106);

a first one-way valve (112) located in said first conduit (100) between said first connector (104) and said third connector (116) permitting fluid flow through said first conduit (100) in a direction from said first connector (104) toward said second connector (106) and preventing fluid flow in the reverse direction; and

a second one-way valve (114) located in said second conduit (102) permitting fluid flow through said second conduit in a direction from said second connector (106) toward said first connector (104) and preventing fluid flow in the reverse direction.

14. The manifold of claim 13 wherein said first and second conduits define an opening (120) between them.

15. The manifold of claim 14 wherein at least one of said first and second conduits has attachment means (122) formed thereon to attach said manifold to a support means.

16. The manifold of claim 15 wherein said attachment means includes a lip (122) formed on at least a portion of said one of said conduits and said lip extends into said opening (120).

**Patentansprüche**

1. Gerät zum Verabreichen radioaktiven Aerosols, umfassend

eine Abstützeinrichtung (40, 42) zum Abstützen einer Erzeugungsquelle (82) für radioaktives Aerosol;

eine Transport- bzw. Fördereinrichtung (88), die mit der genannten Quelle (82) verbindbar ist, um ein radioaktives Aerosol, das von der genannten Quelle erzeugt wurde, zu einem Patienten zu befördern, der in Fluidkommunikation mit der genannten Quelle steht;

und eine Abschirmeinrichtung (30-36), die im wesentlichen die genannte Abstützeinrichtung und Transport- bzw. Fördereinrichtung umgibt, um die Menge an auf die Umgebung übertragener radioaktiver Strahlung zu reduzieren, dadurch gekennzeichnet, daß ein Abschnitt (30) der genannten Abschirmeinrichtung lösbar an der genannten Transport- bzw. Fördereinrichtung (88) befestigt ist und zusammen mit der genannten Transport- bzw. Fördereinrichtung und der genannten Quelle (82) von der genannten Abstützeinrichtung (40, 42) als Einheit entfernbar ist.

2. Gerät nach Anspruch 1, worin die genannte Transport- bzw. Fördereinrichtung eine Sammelleitung (88) einschließlich einer ersten Befestigungseinrichtung (122) umfaßt, die für die lösbare Befestigung an dem genannten Abschnitt (30) der genannten Abschirmeinrichtung ausgebildet ist.

3. Gerät nach Anspruch 2, worin der genannte Abschnitt (30) der genannten Abschirmeinrichtung eine zweite Befestigungseinrichtung (68-76) für den lösbaren Eingriff mit der genannten ersten Befestigungseinrichtung (122) umfaßt.

4. Gerät nach Anspruch 1, 2 oder 3, worin die genannte Sammelleitung als starre Einheit ausgebildet ist und eine erste Leitung (100) und eine zweite Leitung (102) umfaßt, die an einem Ende zusammengefügt sind, um einen ersten Verbinder (104) zu bilden und am anderen Ende zusammengefügt sind, um einen zweiten Verbinder (106) zu bilden; und ein dritter Verbinder (116) in der genannten ersten Leitung (100) zwischen dem genannten ersten und zweiten Verbinder angeordnet und für die Befestigung an der genannten Quelle (82) ausgebildet ist.

5. Gerät nach Anspruch 4, worin die genannte erste und zweite Leitung (100, 102) eine Öffnung (120) zwischen sich begrenzen und eine erste Befestigungseinrichtung (122), die für die lösbare Befestigung an dem genannten Abschnitt (30) der genannten Abschirmeinrichtung ausgebildet ist, auf wenigstens einer der genannten Leitungen vorgesehen ist.

6. Gerät nach Anspruch 5, worin der genannte Abschnitt (30) der genannten Abschirmeinrichtung eine zweite Befestigungseinrichtung (68-76) umfaßt, die dazu ausgebildet ist, in die genannte Öffnung (120) einzudringen und mit der ersten Befestigungseinrichtung (122) in lösbarem Eingriff zu stehen.

7. Gerät nach Anspruch 6, worin die genannte erste Befestigungseinrichtung eine Lippe (122) und die genannte zweite Befestigungseinrichtung wenigstens eine Fläche (72) für den Eingriff mit der genannten Lippe umfaßt.

8. Gerät nach einem der Ansprüche 4 bis 7, umfassend ein erstes Einwegventil (112), das in der genannten ersten Leitung (100) zwischen dem genannten ersten Verbinder (104) und dem genannten dritten Verbinder (116) angeordnet ist und Fluidfluß durch die genannte erste Leitung (100) in einer Richtung vom genannten ersten Verbinder (104) auf den genannten zweiten Verbinder (106) zu ermöglicht und den Fluidfluß in umgekehrter Richtung verhindert, und ein zweites Einwegventil (114), das in der genannten zweiten Leitung (102) angeordnet ist, den Fluidfluß durch die genannte zweite Leitung in einer Richtung weg vom genannten zweiten Verbinder (106) auf den genannten ersten Verbinder (104) zu zuläßt und den Fluidfluß in umgekehrter Richtung verhindert.

9. Gerät nach einem der vorhergehenden Ansprüche, worin die genannte Abschirmeinrichtung einen Abschirmbehälter umfaßt, umfassend:

einen äußeren Mantel (24);

einen inneren Mantel (27), der innerhalb des genannten äußeren Mantels abgestützt ist, wobei der genannte innere Mantel mit einer Innenwand (34) und einer Außenwand (32) ausgebildet ist, die zwischen sich einen Zwischenraum (36) für die Aufnahme von strahlungsabschirmendem Material bilden, wobei die genannte Innenwand (34) einen Abschnitt aufweist, dessen Form allgemein den Konturen einer darin unterzubringenden Quelle von radioaktivem Aerosol und Transport- bzw. Fördereinrichtung entspricht; und

eine entfernbare Abdeckung (30), die mit strahlungsabschirmendem Material ausgebildet ist und einen Abschnitt aufweist, der allgemein den Konturen der darunter in Stellung zu bringenden Transport- bzw. Fördereinrichtung für das radioaktive Aerosol entspricht, wobei die genannte Innenwand und die genannte Abdeckung zwischen sich wenigstens eine Öffnung (54, 56) definieren bzw. begrenzen, um zu ermöglichen, daß die darin untergebrachte Transport-bzw. Fördereinrichtung während der Verwendung in Fluidkommunikation mit der umgebenden Atmosphäre steht.

10. Gerät nach Anspruch 9, worin die genannte Innenwand (34) eine Rampe (52) definiert bzw. begrenzt, die dazu ausgebildet ist, einen Fluidverabreichungsschlauch zur Quelle für radioaktives Aerosol abzustützen.

11. Gerät nach einem der vorhergehenden Ansprüche, umfassend wenigstens eine Öffnung (54, 56), um während der Verwendung die Fluidkommunikation der darin untergebrachten Transport- bzw. Fördereinrichtung mit der umgebenden Atmosphäre zu ermöglichen; und eine strahlungsabschirmende Hilfseinrichtung (26, 45), um die genannte(n) Öffnung(en) (54, 56) zu verschließen, wenn die genannte Quelle und Fördereinrichtung nicht in Verwendung steht, um die genannte Quelle und Fördereinrichtung wirksam von der umgebenden Atmosphäre zu isolieren.

12. Gerät nach Anspruch 11, umfassend eine Abdeckung (22), die dazu ausgebildet ist, von der genannten strahlungsabschirmenden Hilfseinrichtung (26, 45) gehalten zu werden, wenn die genannte strahlungsabschirmende Hilfseinrichtung in Stellung gebracht ist, um die genannte(n) Öffnung(en) (54, 56) zu verschließen.

13. Eine Sammelleitung, die für die Verwendung in der Transport- bzw. Fördereinrichtung des Gerätes nach Anspruch 1 ausgebildet ist und eine erste starre Leitung (100) und eine zweite starre Leitung (102) umfaßt, die an einem Ende zusammengefügt sind, um einen ersten Verbinder (104) zu bilden und am anderen Ende zusammengefÜgt sind, um einen zweiten Verbinder (106) zu bilden;

ein dritter Verbinder (116) ist in der genannten ersten Leitung (100) zwischen dem genannten ersten und zweiten Verbinder (104, 106) angeordnet;

ein erstes Einwegventil (112) ist in der genannten ersten Leitung (100) zwischen dem genannten ersten Verbinder (104) und dem genannten dritten Verbinder (116) angeordnet und ermöglicht den Fluidfluß durch die genannte erste Leitung (100) in einer Richtung vom genannten ersten Verbinder (104) zum genannten zweiten Verbinder (106) und verhindert den Fluidfluß in umgekehrter Richtung; und

ein zweites Einwegventil (114), das in der genannten zweiten Leitung (102) untergebracht ist, läßt Fluidfluß durch die genannte zweite Leitung in einer Richtung vom genannten zweiten Verbinder (106) zum genannten ersten Verbinder (104) zu und verhindert dne Fluidfluß in umgekehrter Richtung.

14. Sammelleitung nach Anspruch 13, worin die genannte erste und zweite Leitung zwischen sich eine Öffnung (120) bilden.

15. Sammelleitung nach Anspruch 14, worin wenigstens entweder die genannte erste oder die genannte zweite Leitung eine darauf ausgebildete Befestigungseinrichtung (122) aufweist, um die genannte Sammelleitung an einer Abstützeinrichtung zu befestigen.

16. Sammelleitung nach Anspruch 15, worin die genannte Befestigungseinrichtung eine Lippe (122) umfaßt, die auf wenigstens einem Abschnitt einer der genannten Leitungen ausgebildet ist und sich in die genannte Öffnung (120) hinein erstreckt.

**Revendications.**

1. Appareil pour l'administration d'un aérosol radioactif comprenant

un moyen de support (40,42) pour supporter une source génératrice d'aérosol radioactif (82);

un moyen de transport (88) pouvant être connecté à ladite source (82) pour transporter un aérosol radioactif produit par ladite source vers un patient en communication de fluide avec ladite source;

et un moyen de blindage (30-36) entourant sensiblement ledit moyen de support et ledit moyen de transport pour réduire la quantité de rayonnement transmis à l'environnement, · caractérisé en ce qu'une portion (30) dudit moyen de blindage est attachée amovible audit moyen de transport (88) et est amovible avec ledit moyen de transport et ladite source (82) dudit moyen de support (40,42) en une unité.

2. Appareil selon la revendication 1, où ledit moyen de transport comprend un collecteur (88) comprenant un premier moyen de fixation (122) adapté à une fixation amovible à ladite portion (30) dudit moyen de blindage.

3. Appareil selon la revendication 2, où ladite portion (30) dudit moyen de blindage comprend un second moyen de fixation (68-76) pour un engagement amovible avec ledit premier moyen de fixation (122).

4. Appareil selon l'une quelconque des revendications 1, 2 ou 3, où ledit collecteur a la forme d'une unité rigide et comprend un premier conduit (100) et un second conduit (102) joints à une extrémité pour former un premier connecteur (104) et à l'autre extrémité pour former un second connecteur (106); et un troisième connecteur (116) placé dans ledit premier conduit (100) entre lesdits premier et second connecteurs et adapté à une fixation à ladite source (82).

5. Appareil selon la revendication 4, où lesdits premier et second conduits (100, 102) définissent une ouverture (120) entre eux et des premiers moyens de fixation (122) permettant la fixation amovible à ladite portion (30) dudit moyen de blindage sont formés sur au moins l'un desdits conduits.

6. Appareil selon la revendication 5, où ladite portion (30) desdits moyens de blindage comprend des seconds moyens de fixation (68-76) adaptés à entrer dans ladite ouverture (120) et à engager de manière amovible lesdits premiers moyens de fixation (122).

7. Appareil selon la revendication 6, où ledit premier moyen de fixation comprend une lèvre (122) et ledit second moyen de fixation comprend au moins une surface (72) pour engager ladite lèvre.

8. Appareil selon l'une quelconque des revendications 4 à 7 comprenant un premier clapet à une voie (112) placé dans ledit premier conduit (100) entre ledit premier connecteur (104) et ledit troisième connecteur (116), permettant un écoulement de fluide à travers ledit premier conduit (100) dans une direction dudit premier connecteur (104) vers ledit second connecteur (106) et empêchant l'écoulement de fluide en direction inverse et un second clapet à une voie (114) placé dans ledit second conduit (102), permettant l'écoulement de fluide à travers ledit second conduit dans une. direction dudit second connecteur (106) vers ledit premier connecteur (104) et empêchant l'écoulement de fluide en direction inverse.

9. Appareil selon l'une quelconque des revendications précédentes, où ledit moyen de blindage comprend un conteneur de blindage comprenant:

une enveloppe externe (24);

une enveloppe interne (27) supportée dans ladite enveloppe externe, ladite enveloppe interne ayant une paroi interne (34) et une paroi externe (32) définissant un espace (36) entre elles pour recevoir un matériau de blindage contre le rayonnement, ladite paroi interne (34) ayant une portion se conformant généralement aux contours d'une source d'un aérosol radioactif et un moyen de transport à y placer; et

un couvercle amovible (30) présentant un matériau de blindage contre le rayonnement et ayant une portion se conformant généralement aux contours du moyen de support de l'aérosol radioactif pour se trouver en dessous, ladite paroi interne et ledit second couvercle définissant au moins une ouverture (54,56) entre eux pour permettre au moyen de transport qui y est placé d'être en communication de fluide avec l'atmosphère environnante lors de son utilisation.

10. Appareil selon la revendication 9, où ladite paroi interne (34) définit une rampe (52) adaptée à supporter un tube d'administration de fluide vers la source d'aérosol radioactif.

11. Appareil selon l'une quelconque des revendications précédentes, où est incorporée au moins une ouverture (54,56) pour permettre au moyen de transport qui y est placé d'être en communication de fluide avec l'atmosphère environnante lorsqu'il est en utilisation; et un moyen de blindage auxiliaire contre le rayonnement (26,45) pour fermer ladite ouverture ou lesdites ouvertures (54,56) lorsque ladite source et le moyen de transport n'est pas en utilisation pour efficacement isoler ladite source et le moyen de transport de l'atmosphère environnante.

12. Appareil selon la revendication 11 comprenant un couvercle (22) pouvant être retenu par lesdits moyens de blindage auxiliaire (26,45) lorsque lesdits moyens de blindage auxiliaire sont placés pour fermer ladite ouverture ou lesdites ouvertures (54,56).

13. Collecteur adapté à une utilisation dans le moyen de transport de l'appareil de la revendication 1, comprenant un premier conduit rigide (100) et un second conduit rigide (102) joints à une extrémité pour former un premier connecteur (104) et à l'autre extrémité pour former un second connecteur (106);

un troisième connecteur (116) placé dans ledit premier conduit (100) entre lesdits premier et second connecteurs (104, 106);

un premier clapet à une voie (112) placé dans ledit premier conduit (100) entre ledit premier connecteur (104) et ledit troisième connecteur (116), permettant un écoulement de fluide à travers ledit premier conduit (100) dans une direction dudit premier connecteur (104) vers ledit second connecteur (106) et empêchant l'écoulement du fluide en direction inverse; et

un second clapet à une voie (114) placé dans ledit second conduit (102), permettant l'écoulement de fluide à travers ledit second conduit dans une direction dudit second connecteur (106) vers ledit premier connecteur (104) et empêchant l'écoulement de fluide en direction inverse.

14. Collecteur selon la revendication 13, où lesdits premier et second conduits définissent une ouverture (120) entre eux.

15. Collecteur selon la revendication 14, où au moins l'un desdits premier et second conduits a un moyen de fixation (122) qui y est formé pour attacher ledit collecteur à un moyen de support.

16. Collecteur selon la revendication 15, où ledit moyen de fixation comprend une lèvre (122) formée sur au moins une portion du premier desdits conduits et ladite lèvre s'étend dans ladite ouverture (120).

FIG. 1

FIG. 2

FIG. 3

FIG. 4

FIG. 6

FIG. 5

FIG. 7

FIG. 9

FIG. 8

FIG. 10

4

FIG. 11

FIG. 12